# EUROPÄISCHE PATENTSCHRIFT

(11) **EP 1 284 954 B1**
(45) Veröffentlichungstag und Bekanntmachung des Hinweises auf die Patenterteilung: **04.08.2004**
(21) Anmeldenummer: 01960249.9
(22) Anmeldetag: 28.05.2001
(51) Int. Cl.: C07C 67/03, C07C 213/06, C07C 219/08, C07D 211/94

(54) **VERFAHREN ZUR HERSTELLUNG VON ESTERN UNGESÄTTIGTER CARBONSÄUREN**
METHOD FOR THE PRODUCTION OF ESTERS OF UNSATURATED CARBOXYLIC ACIDS
PROCEDE DE PRODUCTION D'ESTERS D'ACIDES CARBONIQUES INSATURES

(30) Priorität: 29.05.2000 DE 10026644
(43) Veröffentlichungstag der Anmeldung: 26.02.2003
(73) Patentinhaber: BASF AKTIENGESELLSCHAFT, 67056 Ludwigshafen (DE)
(72) Erfinder: NESTLER, Gerhard, 1070 Wien (AT); SCHRÖDER, Jürgen, 67071 Ludwigshafen (DE)
(74) Vertreter: Pohl, Michael, Dr.
(86) Internationale Anmeldenummer: PCT/EP2001/006079
(87) Internationale Veröffentlichungsnummer: WO 2001/092198

(56) Entgegenhaltungen:
- EP-A- 0 298 867
- EP-A- 0 902 017
- US-A- 3 686 268
- US-A- 4 028 334
- US-A- 5 856 611
- E. YOSHIDA: "Synthesis of poly(eta-caprolactone) with a stable nitroxyl radical as an end-functional group and its application to a counter radical for living radical polymerization" MACROMOLECULES, Bd. 31, Nr. 5, 10. März 1998 (1998-03-10), Seiten 1446-1453, XP000739212

## Beschreibung

Die Erfindung betrifft ein Verfahren zur Herstellung eines Esters einer ungesättigten Carbonsäure, bei dem ein Ester aus der ungesättigten Carbonsäure und einem C₁-C₄-Alkanol in Gegenwart eines Umesterungskatalysators mit einem Alkohol R³OH umgesetzt wird. Die Erfindung betrifft außerdem ein Metallalkoholat und die Verwendung des Metallalkoholats als Umesterungskatalysator in dem erfindungsgemäßen Verfahren.

Unter den Estern ungesättigter Carbonsäuren sind insbesondere die (Meth)acrylsäureester technisch interessant, da sie wertvolle Ausgangsverbindungen zur Herstellung von Polymeren und Copolymeren, die z. B. als Fasern, Kunststoffe, Lacke, Dispersionen oder Klebstoffe Anwendung finden, darstellen. Der Begriff (Meth)acrylsäureester steht für Methacrylsäureester und Acrylsäureester. Die vorliegende Erfindung ist mit besonderem Vorteil zur Herstellung dieser Ester anwendbar und wird nachfolgend am Beispiel von (Meth)acrylsäureestern beschrieben. Sie eignet sich jedoch generell für die Umesterung von niederen Carbonsäureestern, bevorzugt α,β-ungesättigten Carbonsäureestern, und besonders bevorzugt für die Umesterung von Estern ungesättigter Monocarbonsäuren mit 3 bis 6 Kohlenstoffatomen und ungesättigter Dicarbonsäuren mit 4 bis 8 Kohlenstoffatomen.

Die Umesterung von niederen Carbonsäureestern mit höheren Alkoholen zur Herstellung höherer Carbonsäureester in Gegenwart von sauren oder basischen Katalysatoren, und insbesondere die Herstellung von (Meth)acrylsäureestern durch Umesterung in Gegenwart von sauren oder basischen Katalysatoren, ist allgemein bekannt. Allgemein bekannt ist außerdem, dass es sich bei der Umesterungsreaktion um eine Gleichgewichtsreaktion handelt. Um wirtschaftliche Umsätze zu erreichen, muß daher entweder eine der Ausgangssubstanzen in hohem Überschuß eingesetzt oder es muß mindestens eines der Reaktionsprodukte aus dem Gleichgewicht entfernt, d. h. aus dem Reaktionsgemisch laufend abgetrennt werden. Vorzugsweise wird das Produkt mit dem tiefsten Siedepunkt abgetrennt, nämlich das freigesetzte niedere Alkanol, welches in der Regel ein Azeotrop mit dem niederen Carbonsäureester bildet. Demgemäß erfolgt die Umesterung in der Regel in der Weise, dass ein Gemisch aus niederem Carbonsäureester, höherem Alkanol, einem Katalysator und einem Polymerisationsinhibitor oder Inhibitorgemisch zum Sieden erhitzt und das gebildete Azeotrop aus niederem Carbonsäureester und niederem Alkanol über den Kopf einer im Allgemeinen dem Umesterungsreaktor aufgesetzten Destillationskolonne abgetrennt wird.

Dabei ergibt sich eine Reihe von Problemen. Zum einen neigen ungesättigte Carbonsäuren unter dem Einfluß von Hitze oder Licht zur Polymerisation. Vor allem bei der Herstellung und der destillativen Reinigung sind sie Temperaturen ausgesetzt, die leicht eine unerwünschte Polymerisation auslösen können. Die Folge ist eine Verschmutzung der Apparaturen, ein Verstopfen von Leitungen und Pumpen und die Belegung von Kolonnenböden und Wärmetauscherflächen ("fouling"). Das Reinigen der Anlagen ist ein aufwendiger, teurer und umweltbelastender Vorgang, der zudem die Verfügbarkeit der Anlagen stark reduziert.

Ein weiteres Problem ist die Bildung von Nebenprodukten, z. B. von Michael-Additionsprodukten (Addition von Alkanol an die Kohlenstoff-Kohlenstoff-Doppelbindung), von Ethern aus primären Alkoholen und von Olefinen aus sekundären Alkoholen, bedingt durch Dehydratisierung. Die Nebenreaktionen führen zum einen zu Substanzverlusten und machen zum anderen aufwendige Trenn- und Reinigungsverfahren erforderlich.

Viele der zur Verfügung stehenden bekannten Umesterungs-Katalysatoren büßen ihre Aktivität mit der Zeit ein. Manche Katalysatoren sind so stark hydrolyseempfindlich, dass sie ein Arbeiten unter sorgfältigem Wasserausschluß erfordern, oder sie zersetzten sich unter Freisetzung von Bestandteilen, die zusätzliche Reinigungsund Trennverfahren erfordern.

Gängige Katalysatoren sind zur Zeit vor allem Titanalkoholate und zirkoniumalkoholate, deren Alkylgruppen C₁-C₄-Alkylreste darstellen, z. B. Tetramethyl-, Tetraethyl-, Tetraisopropyl-, Tetrapropyl-, Tetraisobutyl- und Tetrabutyltitanat (CH 522 584, CH 464 891, US 2 822 348, EP 298 867, DE 2 319 688, GB 960 005, GB 1 012 817, GB 1 016 042, EP 145 588). Diese Titanate und Zirkonate liefern jedoch keine befriedigenden Ergebnisse, da sie zum Teil thermisch instabil und äußerst hydrolyseempfindlich sind und daher leicht zu störenden Verunreinigungen führen. Außerdem müssen Sie zwecks Abtrennung aus dem Reaktionsgemisch häufig erst zu unlöslichen Produkten hydrolisiert werden (GB 1 012 817, GB 1 016 042, US 2 822 348).

Durch Verwendung von Titantetraisopropylat oder -butylat, den üblichsten Umesterungskatalysatoren, werden beispielsweise Isopropanol bzw. Butanol als Verunreinigungen eingeschleppt, die sich in der Regel in dem abdestillierten, unumgesetzten niederen Ester anreichern. Sie sind aufgrund ihrer Siedepunkte und häufig auch wegen der Bildung von Azeotropen nur schwer zu entfernen und können dann zur Bildung von weiteren Nebenprodukten durch Umesterungsreaktionen oder Addition an die Doppelbindung der Ester führen.

Außerdem ist bekannt, dass Alkyltitanate die Polymerisation fördern und daher zur Bildung von Polymerisat während der Esterherstellung und Aufarbeitung Anlass geben können. Dies führt zur Verringerung der Ausbeute, zu einer Verschmutzung der Apparaturen und zu einer Verkürzung der Anlagenlaufzeiten.

Zur Lösung dieser Probleme wurden verschiedene Vorschläge gemacht. Die CH 464 891 A empfiehlt wegen der Hydrolyseempfindlichkeit der Titanate eine diskontinuierliche Fahrweise bei der Umesterung. Im Gegensatz zur kontinuierlichen Fahrweise können dabei nämlich die Ausgangsprodukte ohne zusätzlich technischen Aufwand entwässert werden. Dies ist aber für die Herstellung im technischen Maßstab nachteilig. EP 118 639 A schlägt die Umesterung mit dem Titanalkoholat des höheren Alkanols in Abwesenheit des höheren Alkanols und in Abwesenheit von Wasser vor, um die Bildung von Azeotropen zu verhindern. Es wird dabei der niedere (Meth)acrylsäureester mit dem Titanalkoholat des höheren Alkanols umgesetzt, wobei neben dem Zielester das Titanat des niederen Alkanols gebildet wird, welches in einem getrennten Reaktionsschritt mit dem höheren Alkanol wieder zu dem entsprechenden Titanat umgesetzt wird. Das Verfahren ist aufwendig und benötigt hohe Titanatmengen. Es hat daher keine wirtschaftliche Bedeutung.

EP 298 867 A beschreibt die Umesterung von Ethylacrylat mit Dialkylaminoalkanolen in Gegenwart von Tetraethyltitanat, um das Einschleusen eines zusätzlichen Alkanols zu vermeiden. Methyl- und Ethyltitanate sind jedoch teuer und gemäß US 3 686 268 als Katalysatoren weniger geeignet.
GB 1 012 817 A schlägt wegen der thermischen Instabilität der Titanate eine Hydrolyse der Titanate bei erhöhter Temperatur vor der destillativen Isolierung des Zielesters vor. Die Bildung von Ablagerungen ("lacquer deposits") in der Destillationsvorrichtung soll dadurch verhindert werden. Da das Verfahren einen technisch aufwendigen Filtrationsschritt aufweist und eine Wiederverwendung des Katalysators nicht ermöglicht, hat es keine wirtschaftliche Bedeutung.

GB 1 016 042 A schlägt zusätzlich die Verwendung eines "Weichmacheröls" vor. Gemäß US 2 822 348 wird der Titanat-Katalysator vor der destillativen Isolierung des Produkts hydrolysiert oder verbleibt in dem Produkt.

zur weitgehenden Vermeidung der Polymerisatbildung wurde der Zusatz von verschiedenen Inhibitoren bzw. Inhibitorgemischen empfohlen, beispielsweise Aminophenole und Aminomethylphenole (EP 145 588), Hydrochinon, Hydrochinonmonomethylether, Phenothiazin, Tertiärbutylcatechin, Methylenblau, Kupfer- bzw. Eisensulfat, allein oder im Gemisch (EP 298 867), Hydrochinon, Hydrochinonmonomethylether, Phenothiazin etc., gegebenenfalls mit einem zusatz von Sauerstoff bzw. Luft (CH 464 891, US 5 037 978). DE 1 067 806 A schlägt zur Vermeidung der Polymerisation vor, die Umesterung unter Druck und bei 180-250°C durchzuführen, um möglichst kurze Verweilzeiten zu erzielen. Derartige Reaktionsbedingungen verlangen technisch aufwendige Apparaturen und sind daher unwirtschaftlich.

DE 1 067 805 A beschreibt die Verwendung von Chinhydron als Inhibitor und die Zugabe eines Teils des zur vollständigen Umesterung notwendigen niederen Acrylsäureesters erst während der Umesterung über die auf dem Reaktor aufgesetzte Kolonne. Das Verfahren ist umständlich und benötigt einen 3-4-fachen Überschuß an niederen Acrylsäureestern.

EP 522 709 A schlägt eine aus mehreren Komponenten bestehende Polymerisationsinhibitor-zusammensetzung vor, die eine N,N'-Dinitrosophenylendiaminverbindung, ein Phenothiazin und gewünschtenfalls zusätzlich ein Hydrochinon, einen Hydrochinonmonomethylether und/oder eine Phenylendiaminverbindung enthält.

US 5 171 888 schlägt als Polymerisationsinhibitor eine Kombination aus einer Phenylendiamin-Verbindung und einer Mangan-Verbindung vor.

Einen weiteren Weg zur Vermeidung polymerer Nebenprodukte wie auch zur Vermeidung von Michael-Additionen und Bildung von Ethern und Olefinen aus Alkanolen zeigen US 5 037 978 und DE 2 805 702 auf. In US 5 037 978 werden Hafniumchelat-Verbindungen und in DE 2 805 702 Chelatverbindungen von Zirkonium und/oder Calcium als Katalysatoren verwendet.

US 3 686 268 beschreibt die Verwendung von Titanphenolaten als Katalysatoren. Diese Katalysatoren schleppen keine unerwünschten Alkanole ein, sollen Hydrolyse-stabil und wiederverwendbar sein und zusätzlich stabilisierend wirken. Die Titanphenolate gemäß US 3 686 268 verringern die Polymerisationsgefahr, jedoch in für die industrielle Umesterung von (Meth)acrylsäureestern nicht ausreichendem Maße. Sie haben deshalb keine praktische Anwendung gefunden. Es besteht daher Bedarf an noch weiter verbesserten Katalysatoren und Verfahren zur Herstellung höherer Ester ungesättigter Carbonsäuren aus ihren niederen Estern.

Aufgabe der vorliegenden Erfindung ist es daher, ein Verfahren zur Herstellung von Estern ungesättigter Carbonsäuren mit höheren Alkoholen aus ihren Estern mit niederen Alkoholen bereitzustellen, bei dem die Bildung unerwünschter Nebenprodukte verringert ist. Eine weitere Aufgabe ist es, die Einschleusung störender Fremdstoffe weitgehend zu vermeiden.

Überraschenderweise wurde nun gefunden, dass diese Aufgaben durch Verwendung eines Metallalkoholats des Titans, Zirkons, Hafniums, Aluminiums, Vanadiums, eines Alkalimetalls oder Erdalkalimetalls gelöst werden, das mindestens eine Gruppe OR¹ aufweist, wobei R¹ ein 2,2,6,6-Tetraalkyl-1-oxylpiperidin-4-Rest ist.

Die Erfindung betrifft somit ein Verfahren zur Herstellung eines Esters einer ungesättigten Carbonsäure durch Umsetzung eines Esters aus der ungesättigten Carbonsäure und einem C₁-C₄-Alkanol mit einem Alkohol R³OH in Gegenwart eines Umesteningskatalysators, wobei R³ für einen C₄-C₂₀-Alkylrest, einen C₅-C₇-Cycloalkylrest, einen Phenyl-C₁-C₄-alkylrest oder einen durch mindestens eine NR⁵R⁵-Gruppe oder durch 1 bis 3 Hydroxyl- oder C₁-C₄-Alkoxygruppen substituierten oder durch ein oder mehrere, insbesondere 1, 2, 3 oder 4, mindestens zwei C-Atome voneinander entfernte Sauerstoffheteroatome unterbrochenen C₂-C₁₂-Alkylrest steht, wobei die Reste R⁵ unabhängig voneinander für C₁-C₆-Alkyl stehen oder zusammen mit dem Stickstoffatom einen 5- bis 7-gliedrigen, heterocyclischen Ring bilden, der gegebenenfalls ein weiteres Stickstoff- oder Sauerstoffheteroatom enthalten kann, wobei als Umesterungskatalysator mindestens ein Metallalkoholat mit mindestens einer Gruppe OR¹ verwendet wird, worin R¹ ein 2,2,6,6-Tetraalkyl-1-oxyl-piperidin-4-Rest ist.

Gegenstand der Erfindung ist außerdem ein Metallalkoholat des Titans, Zirkons, Hafniums, Vanadiums oder eines Erdalkalimetalls, das mindestens eine Gruppe OR¹ aufweist, wobei R¹ ein 2,2,6,6-Tetraalkyl-1-oxylpiperidin-4-Rest ist.

Gegenstand der vorliegenden Erfindung ist weiterhin die Verwendung dieses Metallalkoholats als Veresterungskatalysator, insbesondere als Katalysator für Umesterungen.

Das erfindungsgemäße Verfahren wird bevorzugt in homogener Phase durchgeführt. Die Umsetzung erfolgt nach folgender Gleichung (I), welche die Umesterung am Beispiel von (Meth)acrylsäureestern darstellt: 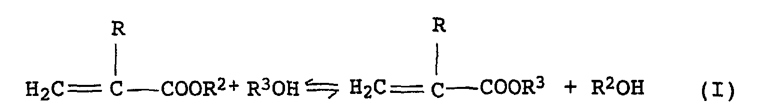 worin R für H oder CH₃ steht, R² für C₁-C₄-Alkyl steht und R³ die oben angegebenen Bedeutungen besitzt.
Das erfindungsgemäße Verfahren ist jedoch nicht nur für Acrylsäureester und Methacrylsäureester geeignet, sondern eignet sich generell für gesättigte und ungesättigte, unsubstituierte und substituierte Mono-, Di- und Polycarbonsäureester, insbesondere aber für Ester α,β-ungesättigter Monocarbonsäuren mit 3 bis 6 Kohlenstoffatomen und α,β-ungesättigter Dicarbonsäuren mit 4 bis 8 Kohlenstoffatomen. Besonders gut geeignet ist das erfindungsgemäße Verfahren für die Umesterung von Estern der Acrylsäure, Methacrylsäure, Crotonsäure, Itaconsäure, Maleinsäure und Fumarsäure.

Die Alkohol-Komponente des Ausgangsesters ist ein Alkanol mit 1 bis 4 Kohlenstoffatomen (R²OH), das verzweigt oder linear sein kann, und besonders bevorzugt sind Methanol und Ethanol.

Der Ausgangsester ist daher bevorzugt ein Methyl-, Ethyl-, n-Propyl-, Isopropyl- oder n-Butylester.

Der Ausgangsalkohol R³OH weist im Allgemeinen einen höheren Siedepunkt und/oder eine größere Kohlenstoffzahl als der freiwerdende Alkohol R²OH auf. R³OH ist bevorzugt ein Alkanol mit 4 bis 20 Kohlenstoffatomen oder ein Alkanol mit 2 bis 12 Kohlenstoffatomen, das mit 1, 2 oder 3 NR⁵R⁵-Gruppen substituiert ist. R⁵ steht insbesondere für gleiche oder verschiedene Alkylreste mit 1 bis 4 Kohlenstoffatomen. Alternativ können die Reste R⁵ zusammen mit dem Stickstoffatom einen 5- bis 7-gliedrigen, gesättigten oder ungesättigten, aromatischen oder nicht-aromatischen heterocyclischen Ring bilden, der gegebenenfalls ein weiteres Heteroatom, insbesondere N oder O, aufweisen kann. Beispiele für derartige Ringe sind Pyrrolidinyl, Piperidinyl, Oxazolidinyl, Morpholinyl, N-Methylpiperazin etc. Vorzugsweise handelt es sich bei dem Ausgangsalkohol R³OH um ein C₆-C₁₈-Alkanol oder ein C₂-C₆-Alkanol, das mit einer NR⁵R⁵-Gruppe substituiert ist, wobei 2-Di-C₁-C₃-alkylaminoethanol und insbesondere 2-Dimethylaminoethanol besonders bevorzugt ist.

Beispiele für geeignete Alkohole R³OH sind n-Butanol, Isobutanol, tert.-Butanol, 2-Ethylhexanol, 2-Propylheptanol, Laurylalkohol, Stearylalkohol, Cyclohexylalkohol, Cyclopentylalkohol, Dimethylaminoethanol, Diethylaminoethanol, 4-Dimethylaminobutan-1-ol, Diethylenglykol, Triethylenglykol, Tetraethylenglykol, 1,4-Butandiol, Trimethylolpropan und Benzylalkohol.

Als Katalysator findet ein Metallalkoholat des Titans, Zirkons, Hafniums, Aluminiums, Vanadiums, eines Alkalimetalls oder Erdalkalimetalls Anwendung, das mindestens eine Gruppe OR¹ aufweist, wobei R¹ ein 2,2,6,6-Tetraalkyl-1-oxyl-piperidin-4-Rest ist. Die Alkylgruppen können gleich oder verschieden sein, und weisen bevorzugt 1 bis 4 Kohlenstoffatome auf. Besonders bevorzugt sind alle 4 Alkylgruppen Methylgruppen.

Außer der mindestens einen Gruppe OR¹ kann das Metallalkoholat eine oder mehrere weitere Alkoholatgruppen enthalten, wobei die Gesamtzahl der Gruppen der Wertigkeit des Metalls entspricht. Die weiteren Alkoholatgruppen können jeweils gleich oder verschieden sein.

Als Metalle sind Titan, Zirkonium, Hafnium, Aluminium, Vanadium und Alkali- und Erdalkalimetalle geeignet, wobei Titan, Zirkonium und Hafnium bevorzugt sind. Am meisten bevorzugt sind Titanalkoholate.

Die bevorzugten erfindungsgemäßen Metallalkoholate haben die folgende Formel (II):

(R¹O)_{b}Me(OR²)_{c}(OR³)_{d} (II)

dabei steht R¹ für den bereits erwähnten 2,2,6,6-Tetraalkyl-1-oxyl-piperidin-4-Rest, und R² und R³ haben die oben angegebene Bedeutung.

Bei einer Wertigkeit a des Metalls steht b für eine ganze Zahl von 1 bis a; c und d stehen jeweils unabhängig voneinander für eine ganze Zahl von 0 bis (a-1), wobei die Summe aus b, c und d der Wertigkeit a des Metalls entspricht.

Besonders bevorzugte Metallalkoholate sind solche, bei denen b = a ist, sowie solche, bei denen d = 0 oder c = 0 ist. Am meisten bevorzugt sind Titan-, Zirkonium-, Hafnium-Alkoholate mit ein bis vier 2,2,6,6-Tetramethyl-1-oxyl-piperidin-4-Resten, und unter diesen das Titanat mit vier 2,2,6,6-Tetramethyl-1-oxyl-piperdin-4-Resten.

Die Metallalkoholate gemäß der vorliegenden Erfindung können alleine oder als Gemisch mehrerer erfindungsgemäßer Metallalkoholate als Umesterungs-Katalysatoren eingesetzt werden.

Die Herstellung der erfindungsgemäßen Katalysatoren kann in an sich bekannter Weise durch Umesterung erfolgen, wie beispielsweise beschrieben in US-A-3 686 268, auf deren Inhalt in vollem Umfang Bezug genommen wird. Es gilt folgende allgemeine Reaktionsgleichung (III): 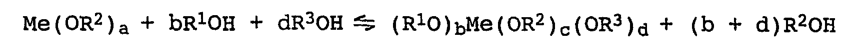 wobei Me, R¹ bis R³, a, b, c, und d die oben angegebenen Bedeutungen besitzen.

Die Herstellung erfolgt im Allgemeinen in der Weise, dass das Ausgangs-Metallalkoholat mit der entsprechenden 4-Hydroxy-2,2,6,6-tetraalkylpiperidin-1-oxyl-Verbindung und gegebenenfalls einem weiteren Alkohol im gewünschten molaren Verhältnis in Gegenwart oder in Abwesenheit eines inerten Lösungsmittels erhitzt wird. Der weitere Alkohol ist vorzugsweise der dem Zielester der Umesterungsreaktion, für die das Metallalkoholat eingesetzt werden soll, zugrundeliegende Alkohol. Die dem Ausgangs-Metallalkoholat zugrundeliegende Alkoholkomponente wird freigesetzt und aus dem Reaktionsgemsich entfernt, z. B. durch Abdestillieren.

Die erfindungsgemäßen Metallalkoholate können außerdem auf bekannte Weise aus den entsprechenden Halogeniden der Metalle Me hergestellt werden, siehe z. B. Houben-Weyl, Methoden der Organischen Chemie, Bd. 6/2, Seite 1 bis 70 (1963).

Das erfindungsgemäße Umesterungsverfahren kann sowohl diskontinuierlich als auch kontinuierlich durchgeführt werden. Bei der kontinuierlichen Umesterung läßt man beispielsweise den Ausgangsester, den Ausgangsalkohol, den Katalysator und ggf. weitere Komponenten, wie Inhibitoren, einzeln oder als Gemisch in den Umesterungsreaktor oder eine ggf. auf den Reaktor aufgesetzte Destillationskolonne einlaufen. Man verwendet einen üblichen Umesterungsreaktor, der gegebenenfalls mit einer Rührvorrichtung und mit einer Heizvorrichtung zur Zuführung der benötigten Reaktionswärme ausgestattet ist. Der im Laufe der Umesterung freiwerdende niedere Alkohol wird aus dem Reaktionsgemisch entfernt, z. B. durch Abdestillieren. Häufig wird der freiwerdende Alkohol als azeotropes Gemisch mit dem Ausgangsester aus dem Reaktionsgemisch entfernt. Als Rückstand bleibt der gebildete Zielester ggf. zusammen mit unumgesetztem Ausgangsester. Der Rückstand (Sumpfprodukt) wird der weiteren Aufarbeitung zugeführt. Im Allgemeinen erfolgt eine Fraktionierung des Sumpfproduktes in einer zweiten Destillationskolonne. Der Zielester wird dabei als Destillat gewonnen. Als Rückstand bleibt der Katalysator, gegebenenfalls zusammen mit dem verwendeten Inhibitor,und Polymerisationsprodukten. Der Rückstand kann teilweise oder vollständig in den Reaktor zurückgeführt werden. Das abdestillierte Gemisch (Leichtsieder) kann in den Reaktor zur Herstellung des Ausgangsesters zurückgeführt werden.

Vorrichtungen zur Durchführung von Umesterungsverfahren sind allgemein bekannt. Eine brauchbare Vorrichtung ist beispielsweise beschrieben in DE 23 19 688. Diese Druckschrift beschreibt auch eine geeignete Verfahrensführung.

Bei dem erfindungsgemäßen Verfahren werden Ausgangsalkohol und Ausgangsester in einem Molverhältnis von 2:1 bis 1:10, bevorzugt 1:1 bis 5 , eingesetzt. Die eingesetzte Katalysatormenge beträgt bevorzugt 0,1 bis 10 Gew.-%, besonders bevorzugt 0,5 bis 3 Gew.-% des Reaktionsgemisches aus Ausgangsester und Ausgangsalkohol. Die Reaktion wird bei üblichen Umesterungstemperaturen durchgeführt, bevorzugt bei etwa 80 bis 120°C, je nach Reaktionspartnern. Der bevorzugte Reaktionsdruck ist Atmosphärendruck, und die Reaktionsdauer beträgt in der Regel etwa 1 bis 10 Stunden.

Die erfindungsgemäßen Metallalkoholat-Katalysatoren wirken stabilisierend und daher ist es möglich, ohne zusätzlichen Inhibitor zu arbeiten. Je nach Reaktionsteilnehmern und Reaktionsbedingungen kann es jedoch sinnvoll sein, einen Inhibitor oder ein Inhibitorgemisch zuzugeben. In Frage kommen dafür z. B. die bekannten Polymerisationsinhibitoren wie Hydrochinon, Hydrochinonmonomethylether, Di-tert-butyl-brenzkatechin, Phenothiazin, p-Phenylendiamine, Methylenblau und Indolin, allein oder als Gemisch von zwei oder mehreren Inhibitoren. Der bevorzugte Inhibitor ist Phenothiazin. Inhibitoren werden in einer Menge von bevorzugt 0,001 bis 1 Gew.-% des Reaktionsgemisches eingesetzt.

Aufgrund der stabilisierenden Wirkung der erfindungsgemäßen Katalysatoren wird die Polymerisatbildung, die bei der Umesterung ungesättigter Carbonsäuren ein permanentes Problem darstellt, weitgehend verhindert. Die verwendeten Apparaturen bleiben über einen langen Zeitraum frei von polymeren Rückständen, so dass Ausfallzeiten durch anfallende Reinigungsarbeiten vermieden werden können. Auch Substanzverluste durch unerwünschte Nebenreaktionen werden weitgehend vermieden. Bei Verwendung der erfindungsgemäßen Katalysatoren ist es nicht erforderlich, wasserfrei zu arbeiten, da sie sehr hydrolysebeständig sind. In Verbindung mit ihrer ausgezeichneten Temperaturbeständigkeit garantiert dies, dass keine störenden Fremdstoffe, die unter Aufwand von Zeit und Kosten abgetrennt werden müßten, eingeschleust werden. Die erfindungsgemäßen Alkoholat-Katalysatoren bleiben im Laufe der Umesterungsreaktion im Wesentlichen unverändert hinsichtlich Zusammensetzung und Aktivität und können immer wieder verwendet werden. Ihre Abtrennung aus den Reaktionsprodukten ist unproblematisch und kann durch einfaches Abdestillieren des Zielesters und eventuell vorhandener sonstiger leichter flüchtiger Komponenten erfolgen. Das Metallalkoholat verbleibt im Destillationsrückstand, der ansonsten im Wesentlichen aus polymeren Nebenprodukten besteht. Da nur sehr wenig polymere Nebenprodukte gebildet werden ist in der Regel keine weitere Auftrennung erforderlich, sondern das Metallalkoholat kann ohne weitere Reinigung in die Reaktion zurückgeführt werden bzw. für eine neue Umesterungs-Charge eingesetzt werden.

Im folgenden wird die Erfindung anhand von Beispielen erläutert, ohne sie zu begrenzen.

### Beispiel 1

Polymerisation von n-Butylacrylat

In einem 500 ml Rundkolben aus Glas wurden 300 g n-Butylacrylat, stabilisiert mit 15 ppm Hydrochinonmonomethylether, auf 100°C erhitzt und bei dieser Temperatur gehalten. Die Temperatur des Kolbeninhalts wurde kontinuierlich gemessen und mit Hilfe eines Schreibers der Verlauf aufgezeichnet. Bei Eintritt der Polymerisation trat ein deutlicher Temperatursprung auf. Die Zeit bis zur Polymerisation wurde bestimmt (Induktionszeit). Ebenso wurde mit einem Gemisch aus dem stabilisierten n-Butylacrylat und 0,5% Titantetraisopropylat verfahren.
Die Induktionszeit bei Butylacrylat betrug 43 Stunden, nach dem Zusatz des Titanates lediglich 30 Stunden.

### Beispiel 2

Es wurden entsprechend Beispiel 1 die Induktionszeiten bei Zusatz von 15 ppm des Titanats des Hydrochinonmonomethylethers (US 3 686 268) und des 4-Hydroxy-2,2,6,6 tetramethylpiperidin-N-oxyls (s. Beispiel 3) bestimmt. Sie betrugen 54 bzw. 100 Stunden.

Wie aus den Beispielen 1 und 2 ersichtlich ist, verringert der Titantetraisopropylat-Katalysator die Induktionszeit der Butylacrylat-Polymerisation erheblich, während der Katalysator auf Basis des Hydrochinonmonomethylethers die Induktionszeit erhöht. Das bei weitem beste Ergebnis wird jedoch bei Zusatz des erfindungsgemäßen Katalysators erzielt. Die Gefahr der Polymerisation während der Umesterung ist somit bei Verwendung der erfindungsgemäßen Katalysatoren erheblich verringert.

### Beispiel 3

Herstellung von Tetra-2,2,6,6-tetramethylpiperidin-N-oxyl-4-titanat

Ein Gemisch aus Isopropyltitanat und 4-Hydroxy-2,2,6,6-tetramethyl-piperidin-N-oxyl (molar 1:4) wurde 3 Stunden auf 80°C erhitzt und anschließend im Vakuum (20 mbar) das gebildete Isopropanol abdestilliert.
Der Rückstand, der in der Kälte erstarrte, enthielt entsprechend der Elementaranalyse 6,6% Ti (Theorie 6,54%), 7,61% N (Theorie: 7,65%), 58,9%C (Theorie: 59,01%) und 9,5% H (Theorie 9,29%).

### Beispiel 4

Herstellung von N,N-Dimethylaminoethylacrylat aus Ethylacrylat

In einem Rührreaktor mit aufgesetzter Destillationskolonne (100 cm, 0,5 cm Raschigringe) und Kondensator wurde ein Gemisch aus 223 g Dimethylaminoethanol, 500 g Ethylacrylat (0,02 Gew.-% H₂O-Gehalt), 20,3 g Katalysator aus Beispiel 3 und 0,01 g Phenothiazin unter Rühren und unter Normaldruck zum Sieden erhitzt. Die Reaktionstemperatur stieg innerhalb der Reaktionszeit (3,5 Stunden) von 105°C auf 130°C an. Über den Kopf der Kolonne wurde ein Gemisch aus Ethanol und Ethylacrylat abgezogen, wobei das Rücklaufverhältnis zuerst 1:1 betrug und nach 3 Stunden auf 5:1 erhöht wurde. Es wurden 230 g Destillat abgetrennt. Nach Beendigung der Umesterung wurde das Reaktionsgemisch einer einfachen Vakuumdestillation (25 mbar, 120°C Endtemperatur) unterworfen. Es wurden 475 g Destillat (349 g Dimethylaminoethylacrylat, 126 g Ethylacrylat) gewonnen. Der Destillationsrückstand, hauptsächlich Katalysator, Inhibitor und Polymere, betrug 28 g. Entsprechend der gaschromatographischen Analyse des Destillats wurde, bezogen auf den Alkohol, eine Ausbeute von 97,5% erreicht.

### Beipiel 5

Es wurde wie unter Beispiel 4 verfahren, als Katalysator wurden jedoch 7,8 g Isopropyltitanat verwendet. Der Rückstand betrug 48 g und die entsprechende Ausbeute 94%.

### Beispiel 6

Beispiel 4 wurde wiederholt, jedoch wurde als Katalysator der Rückstand aus Beispiel 4 eingesetzt. Die Umsetzung verlief entsprechend dem Beispiel 4. Als Destillationsrückstand fielen 30 g an.

Wie aus den Beispielen 4,5 und 6 ersichtlich ist, fallen bei Verwendung des erfindungsgemäßen Katalysators deutlich weniger polymere Rückstände an als bei Verwendung des konventionellen Isopropyltitanat-Katalysators. Der erfindungsgemäße Katalysator kann ohne Abtrennung von den polymeren Reaktionsprodukten direkt wieder für eine erneute Reaktion verwendet werden.

### Beispiel 7

Herstellung von Tetra-2,2,6,6-tetramethylpiperidin-N-oxyl-4-zirkonat

Ein Gemisch aus Zirkon-n-propylat und 4-Hydroxy-2,2,6,6-tetramethyl-piperidin-N-oxyl (molar 1:4) wurde 3 Stunden auf 80°C erhitzt und anschließend wurde im Vakuum (20 mbar) das gebildete Propanol abdestilliert.
Der Rückstand enthielt entsprechend der Elementaranalyse
11,8% Zr (Theorie 11,71%), 7,0% N (Theorie: 7,19 %), 55,5 % C (Theorie: 55,45 %).

### Beispiel 8

Herstellung von Dimethylaminoethyl-tris-2,2,6,6-tetramethylpiperidin-N-oxyl-4-titanat

Ein Gemisch aus Tetramethyltitanat, 4-Hydroxy-2,2,6,6-tetramethyl-piperidin-N-oxyl und Dimethylaminoethanol (molar 1:3:1) wurde 3 Stunden auf 80°C erhitzt und das gebildete Methanol abdestilliert.
Der Rückstand enthielt entsprechend der Elementaranalyse 7,6% Ti
(Theorie 6,4%), 8,4% N (Theorie: 8,6%), 57,3 % C
(Theorie: 57,06%).

### Beispiel 9

Herstellung von N,N-Dimethylaminoethylacrylat

Es wurde wie in Beispiel 4 verfahren, als Katalysator wurden jedoch 20 g Dimethylaminoethyl-tris-2,2,6,6-tetramethylpiperidin-N-oxyl-4-titanat verwendet.

Der Rückstand betrug 29 g und die Ausbeute war 97%.

## Patentansprüche

1. Verfahren zur Herstellung eines Esters einer ungesättigten Carbonsäure durch Umsetzung eines Esters aus der ungesättigten Carbonsäure und einem C₁-C₄-Alkanol mit einem Alkohol R³OH in Gegenwart eines Umesterungskatalysators, wobei R³ für einen C₄-C₂₀-Alkylrest, einen C₅-C₇-Cycloalkylrest, einen Phenyl-C₁-C₄-alkylrest oder einen durch mindestens eine NR⁵R⁵-Gruppe oder durch 1 bis 3 Hydroxylgruppen oder C₁-C₄-Alkoxylgruppen substituierten oder durch ein oder mehrere Sauerstoffheteroatome unterbrochenen C₂-C₁₂-Alkylrest steht, wobei die Reste R⁵ unabhängig voneinander für C₁-C₆-Alkyl stehen oder zusammen mit dem Stickstoffatom einen 5- bis 7-gliedrigen, heterocyclischen Ring bilden, der gegebenenfalls ein weiteres Stickstoff- oder Sauerstoffheteroatom enthalten kann, **dadurch gekennzeichnet, dass** als Umesterungskatalysator mindestens ein Metallalkoholat des Titans, Zirkons, Hafniums, Aluminiums, Vanadiums, eines Alkalimetalls oder Erdalkalimetalls mit mindestens einer Gruppe OR¹ verwendet wird, wobei R¹ ein 2,2,6,6-Tetraalkyl-1-oxyl-piperidin-4-Rest ist.

2. Verfahren nach Anspruch 1, **dadurch gekennzeichnet, dass** die ungesättigte Carbonsäure eine α,β-ethylenisch ungesättigte Monocarbonsäure mit 3 bis 6 Kohlenstoffatomen oder eine Dicarbonsäure mit 4 bis 8 Kohlenstoffatomen ist.

3. Verfahren nach Anspruch 1 oder 2, **dadurch gekennzeichnet, dass** die ungesättigte Carbonsäure Acrylsäure oder Methacrylsäure ist.

4. Verfahren nach einem der vorhergehenden Ansprüche, wobei das Metallalkoholat ausgewählt ist unter Metallalkoholaten des Titans, des Zirkons und des Hafniums.

5. Verfahren nach einem der vorhergehenden Ansprüche, **dadurch gekennzeichnet, dass** man einen Alkohol R³OH verwendet, wobei R³ für einen C₂-C₆-Alkylrest steht, der mit einer NR⁵R⁵-Gruppe substituiert ist und die Reste R⁵ unabhängig voneinander für C₁-C₃-Alkyl stehen.

6. Verfahren nach einem der vorhergehenden Ansprüche, **dadurch gekennzeichnet, dass** als Umesterungskatalysator mindestens ein Metallalkoholat der Formel (II) verwendet wird
(R¹O)_{b}Me(OR²)_{c}(OR³)_{d} (II)
in der
R¹ für einen 2,2,6,6-Tetraalkyl-1-oxyl-piperidin-4-Rest steht,
R² für einen C₁-bis C₄-Alkylrest steht,
R³ für einen C₄-C₂₀-Alkylrest, einen C₅-C₇-Cycloalkylrest, einen Phenyl-C₁-C₄-alkylrest oder einen C₂-C₁₂-Alkylrest, der durch mindestens eine NR⁵R⁵-Gruppe oder durch 1 bis 3 Hydroxylgruppen oder C₁-C₄-Alkoxylgruppen substituiert oder durch ein oder mehrere Sauerstoffheteroatome unterbrochen ist, wobei die Reste R⁵ unabhängig voneinander für C₁-C₆-Alkyl stehen oder zusammen mit dem Stickstoffatom einen 5- bis 7-gliedrigen, heterocyclischen Ring bilden, der gegebenenfalls ein weiteres Stickstoff- oder Sauerstoffheteroatom enthalten kann, steht,
Me für Ti, Zr, Hf, Al, V, ein Alkali- oder Erdalkalimetall steht,
b für eine ganze Zahl von 1 bis a steht,
c und d unabhängig voneinander für eine ganze Zahl von 0 bis (a-1) stehen, wobei
a die Wertigkeit des Metalls ist und die Beziehung:
b + c + d = a gilt.

7. Verfahren nach Anspruch 6, **dadurch gekennzeichnet, dass** man das Metallalkoholat in einer Menge von 0,1 bis 10 Gew.-%, bezogen auf das Reaktionsgemisch aus Ester und Alkohol R³OH, verwendet.

8. Metallalkoholat des Titans, Zirkoniums, Hafniums, Vanadiums oder eines Erdalkalimetalls, das mindestens eine Gruppe OR¹ aufweist, wobei R¹ ein 2,2,6,6,-Tetraalkyl-1-oxyl-piperidin-4-Rest ist.

9. Metallalkoholat nach Anspruch 8, des Titans, des Zirkoniums oder des Hafniums.

10. Metallalkoholat nach Anspruch 8 der Formel (II):
(R¹O)_{b}Me(OR²)_{c}(OR³)_{d} (II)
in der
R¹ für einen 2,2,6,6-Tetraalkyl-1-oxyl-piperidin-4-Rest steht,
R² für einen C₁-bis C₄-Alkylrest steht,
R³ für einen C₄-C₂₀-Alkylrest, einen C₅-C₇-Cycloalkylrest, einen Phenyl-C₁-C₄-alkylrest oder einen C₂-C₁₂-Alkylrest, der durch mindestens eine NR⁵R⁵-Gruppe oder durch 1 bis 3 Bydroxylgruppen oder C₁-C₄-Alkoxylgruppen substituiert oder durch ein oder mehrere Sauerstoffheteroatome unterbrochen ist, wobei die Reste R⁵ unabhängig voneinander für C₁-C₆-Alkyl stehen oder zusammen mit dem Stickstoffatom einen 5- bis 7-gliedrigen, heterocyclischen Ring bilden, der gegebenenfalls ein weiteres Stickstoff- oder Sauerstoffheteroatom enthalten kann, steht,
Me für Ti, Zr, Hf,oder ein Erdalkalimetall steht,
b für eine ganze Zahl von 1 bis a steht,
c und d unabhängig voneinander für eine ganze Zahl von 0 bis (a-1) stehen, wobei
a die Wertigkeit des Metalls ist und die Beziehung:
b + c + d = a gilt.

11. Metallalkoholat nach Anspruch 10 der Formel (II), worin He für Ti oder Zr steht,
R¹ für den 2,2,6,6-Tetramethyl-1-oxyl-pipexidin-4-Rest steht, und
b für 4 und c und d für 0 stehen.

12. Verwendung eines Metallalkoholats nach einem der Ansprüche 8 bis 11 als Katalysator bei der Umesterung eines Esters einer ungesättigten Carbonsäure.

## Claims

1. A process for preparing an ester of an unsaturated carboxylic acid by reacting an ester of the unsaturated carboxylic acid and a C₁-C₄-alkanol with an alcohol R³OH, where R³ is a C₄-C₂₀-alkyl radical, a C₅-C₇-cycloalkyl radical, a phenyl-C₁-C₄-alkyl radical or a C₂-C₁₂-alkyl radical substituted by at least one NR⁵R⁵ group or by from 1 to 3 hydroxyl groups or C₁-C₄-alkoxyl groups or interrupted by one or more oxygen atoms, where the radicals R⁵ are, independently of one another, C₁-C₆-alkyl or together with the nitrogen atom form a 5- to 7-membered heterocyclic ring which may contain a further nitrogen or oxygen atom, in the presence of a transesterification catalyst comprising at least one metal alkoxide of titanium, zirconium, hafnium, aluminium, vanadium of an alkali metal or of an alkaline earth metal containing at least one OR¹ group, where R¹ is a 2,2,6,6-tetraalkyl-1-oxylpiperidin-4-yl radical.

2. A process as claimed in claim 1, wherein the unsaturated carboxylic acid is an α,β-ethylenically unsaturated monocarboxylic acid having from 3 to 6 carbon atoms or a dicarboxylic acid having from 4 to 8 carbon atoms.

3. A process as claimed in claim 1 or 2, wherein the unsaturated carboxylic acid is acrylic acid or methacrylic acid.

4. A process as claimed in any of the preceding claims, wherein the metal alkoxide is selected from metal alkoxides of titanium, zirconium or hafnium.

5. A process as claimed in any of the preceding claims, wherein the alcohol R³OH used is one in which R³ is a C₂-C₆-alkyl radical which is substituted by an NR⁵R⁵ group and the radicals R⁵ are, independently of one another, C₁-C₃-alkyl.

6. A process as claimed in any of the preceding claims, wherein the transesterification catalyst used comprises at least one metal alkoxide of the formula (II)
(R¹O)_{b}Me(OR²)_{c}(OR³)_{d} (II)
where
R¹ is a 2,2,6,6-tetraalkyl-1-oxylpiperidin-4-yl radical,
R² is a C₁-C₄-alkyl radical,
R³ is a C₄-C₂₀-alkyl radical, a C₅-C₇-cycloalkyl radical, a phenyl-C₁-C₄-alkyl radical or a C₂-C₁₂-alkyl radical which is substituted by at least one NR⁵R⁵ group or by from 1 to 3 hydroxyl groups or C₁-C₄-alkoxyl groups or interrupted by one or more oxygen atoms, where the radicals R⁵ are,
independently of one another, C₁-C₆-alkyl or together with the nitrogen atom form a 5- to 7-membered heterocyclic ring which may contain a further nitrogen or oxygen atom,
Me is Ti, Zr, Hf, Al, V, an alkali metal or an alkaline earth metal,
b is an integer from 1 to a,
c and d are each, independently of one another, an integer from 0 to (a-1), where
a is the valence of the metal and
b + c + d = a.

7. A process as claimed in claim 6, wherein the metal alkoxide is used in an amount of from 0.1 to 10% by weight, based on the reaction mixture of ester and alcohol R³OH.

8. A metal alkoxide of titanium, zirconium, hafnium, vanadium or of an alkaline earth metal containing at least one OR¹ group, where R¹ is a 2,2,6,6-tetraalkyl-1-oxylpiperidin-4-yl radical.

9. A metal alkoxide as claimed in claim 8 of titanium, zirconium or hafnium.

10. A metal alkoxide as claimed in claim 8 having the formula (II):
(R¹O)_{b}Me(OR²)_{c}(OR³)_{d} (II)
where
R¹ is a 2,2,6,6-tetraalkyl-1-oxylpiperidin-4-yl radical,
R² is a C₁-C₄-alkyl radical,
R³ is a C₄-C₂₀-alkyl radical, a C₅-C₇-cycloalkyl radical, a phenyl-C₁-C₄-alkyl radical or a C₂-C₁₂-alkyl radical which is substituted by at least one NR⁵R⁵ group or by from 1 to 3 hydroxyl groups or C₁-C₄-alkoxyl groups or interrupted by one or more oxygen atoms, where the radicals R⁵ are,
independently of one another, C₁-C₆-alkyl or together with the nitrogen atom form a 5- to 7-membered heterocyclic ring which may contain a further nitrogen or oxygen atom,
Me is Ti, Zr, Hf or an alkaline earth metal,
b is an integer from 1 to a,
c and d are each, independently of one another, an integer from 0 to (a-1), where
a is the valence of the metal and
b + c + d = a.

11. A metal alkoxide as claimed in claim 10 having the formula (II), in which Me is Ti or Zr,
R¹ is the 2,2,6,6-tetramethyl-1-oxylpiperidin-4-yl radical and b is 4 and c and d are each 0.

12. The use of a metal alkoxide as claimed in any of claims 8 to 11 as catalyst in the transesterification of an ester of an unsaturated carboxylic acid.

## Revendications

1. Procédé de préparation d'un ester d'un acide carboxylique insaturé par réaction d'un ester de l'acide carboxylique insaturé et d'un alcanol en C₁-C₄ avec un alcool R³OH en présence d'un catalyseur de transestérification, où R³ représente un reste alkyle en C₄-C₂₀, un reste cycloalkyle en C₅-C₇, un reste phényl(alkyle en C₁-C₄) ou un reste alkyle en C₂-C₁₂, substitué par au moins un radical NR⁵R⁵ ou par 1 à 3 radicaux hydroxyle ou par un radical alcoxyle C₁-C₄ ou interrompu par un ou plusieurs hétéroatomes oxygène, où les restes R⁵ représentent indépendamment l'un de l'autre, alkyle en C₁-C₆, ou forment avec l'atome d'azote, un hétérocycle ayant 5 à 7 membres, qui peut contenir le cas échéant un autre hétéroatome azote ou oxygène, **caractérisé en ce que** comme catalyseur de transestérification, on utilise au moins un alcoolate métallique du titane, du zirconium, du hafnium, de l'aluminium, du vanadium, d'un métal alcalin ou d'un métal alcalino-terreux avec au moins un radical OR¹, où R¹ est un reste 2,2,6,6-tétraalkyl-1-oxypipéridin-4-yle.

2. Procédé selon la revendication 1, **caractérisé en ce que** l'acide carboxylique insaturé est un acide monocarboxylique α,β-éthyléniqument insaturé ayant 3 à 6 atomes de carbone ou un diacide carboxylique ayant 4 à 8 atomes de carbone.

3. Procédé selon la revendication 1 ou 2, **caractérisé en ce que** l'acide carboxylique insaturé est l'acide acrylique ou l'acide méthacrylique.

4. Procédé selon l'une des revendications précédentes, où l'alcoolate métallique est choisi parmi les alcoolates métalliques du titane, du zirconium et du hafnium.

5. Procédé selon l'une des revendications précédentes **caractérisé en ce que** l'on utilise un alcool R³OH, où R³ représente un reste alkyle en C₂-C₆, qui est substitué par un radical NR⁵R⁵ et les restes R⁵ représentent indépendamment l'un de l'autre, alkyle en C₁-C₃.

6. Procédé selon l'une des revendications précédentes **caractérisé en ce que** l'on utilise comme catalyseur de transestérification, au moins un alcoolate métallique de la formule (II) :
(R¹O)_{b}Me(OR²)_{c}(OR³)_{d} (II)
dans laquelle:
R¹ représente un reste 2,2,6,6-tétraalkyl-1-oxypipéridin-4-yle,
R² représente un reste alkyle en C₁-C₄,
R³ représente un reste alkyle en C₄-C₂₀, un reste cycloalkyle en C₅-C₇, un reste phényl(alkyle en C₁-C₄) ou un reste alkyle en C₂-C₁₂, substitué par au moins un radical NR⁵R⁵ ou par 1 à 3 radicaux hydroxyle ou par un radical alcoxyle C₁-C₄ ou interrompu par un ou plusieurs hétéroatomes oxygène, où les restes R⁵ représentent indépendamment l'un de l'autre, alkyle en C₁-C₆, ou forment avec l'atome d'azote, un hétérocycle ayant 5 à 7 membres, qui peut contenir le cas échéant un autre hétéroatome azote ou oxygène,
Me représente Ti, Zr, Hf, Al, V, un métal alcalin ou un métal alcalino-terreux,
b représente un nombre entier allant de 1 à a,
c et d représentent indépendamment l'un de l'autre, un nombre entier allant de 0 à (a-1), où
a est la valence du métal et où l'on a :
b + c + d = a.

7. Procédé selon la revendication 6, **caractérisé en ce que** l'on utilise l'alcoolate métallique en une quantité allant de 0,1 à 10% en poids, sur base du mélange réactionnel constitué de l'ester et de l'alcool R³OH.

8. Alcoolate métallique du titane, du zirconium, du hafnium, du vanadium ou d'un métal alcalino-terreux, qui présente au moins un radical OR¹, où R¹ est un reste 2,2,6,6-tétraalkyl-1-oxypipéridin-4-yle.

9. Alcoolate métallique selon la revendication 8, du titane, du zirconium ou du hafnium.

10. Alcoolate métallique selon la revendication 8, de la formule (II) :
(R¹O)_{b}Me(OR²)_{c}(OR³)_{d} (II)
dans laquelle:
R¹ représente un reste 2,2,6,6-tétraalkyl-1-oxypipéridin-4-yle,
R² représente un reste alkyle en C₁-C₄,
R³ représente un reste alkyle en C₄-C₂₀, un reste cycloalkyle en C₅-C₇, un reste phényl(alkyle en C₁-C₄) ou un reste alkyle en C₂-C₁₂, substitué par au moins un radical NR⁵R⁵ ou par 1 à 3 radicaux hydroxyle ou par un radical alcoxyle C₁-C₄ ou interrompu par un ou plusieurs hétéroatomes oxygène, où les restes R⁵ représentent indépendamment l'un de l'autre, alkyle en C₁-C₆, ou forment avec l'atome d'azote, un hétérocycle ayant 5 à 7 membres, qui peut contenir le cas échéant un autre hétéroatome azote ou oxygène,
Me représente Ti, Zr, Hf ou un métal alcalino-terreux,
b représente un nombre entier allant de 1 à a,
c et d représentent indépendamment l'un de l'autre, un nombre entier allant de 0 à (a-1), où
a est la valence du métal et où l'on a :
b + c + d = a.

11. Alcoolate métallique selon la revendication 10, de la formule (II), où Me représente Ti ou Zr,
R¹ représente un reste 2,2,6,6-tétraméthyl-1-oxypipéridin-4-yle,
b est 4 et c et d représentent 0.

12. Utilisation d'un alcoolate métallique selon une des revendications 8 à 11, comme catalyseur dans la transestérification d'un ester d'un acide carboxylique insaturé.
